# EUROPEAN PATENT APPLICATION

(11) **EP 4 597 443 A1**
(43) Date of publication of application: **06.08.2025**
(21) Application number: 25155608.0
(22) Date of filing: 03.02.2025
(51) Int. Cl.: G06V 10/26, G06V 10/46, G06V 10/48, G06V 10/764, G06V 20/69, G06T 7/10, G06T 7/60

(54) **APPARATUS FOR PERFORMING SERUM-AGGLUTINATION TESTS**

(30) Priority: 05.02.2024 IT 202400002295
(71) Applicant: Diesse Diagnostica Senese S.p.a., 20157 Milano (IT)
(72) Inventor: NORELLI, Michele, 53035 Monteriggioni (SI) (IT); BERRETTI, Mirko, 53035 Monteriggioni (SI) (IT); MASSARI, Thomas, 53035 Monteriggioni (SI) (IT)
(74) Representative: Botti & Ferrari S.p.A.

(57) **Abstract**

An apparatus for performing serum-agglutination tests is described, the apparatus having a housing area for housing a plate with a plurality of wells containing a sample to be analyzed, a processing unit, and at least one image detector in communication with the processing unit for acquiring images of the plate located in the housing area. The processing unit comprises instructions for processing the acquired images which, when executed, cause said processing unit to carry out the following steps: controlling the acquisition of at least one image containing at least one well, extracting a plurality of features from the image, said features comprising at least one number of aggregates of pixels detectable in said image, a number of closed contours detectable in said image, and a number of elements having a determined at least partially circular and/or elliptical geometry detectable in said image; and classifying, based on a combination of said features, the sample contained in the well, providing information on the positivity of said sample through said classification.

## Description

### Field of application

The present invention refers to an apparatus for performing serum-agglutination tests in a microplate, for example for the identification of infectious diseases. The following description refers to this field of application with the only purpose to simplify its exposition.

### Prior art

As known, serum-agglutination tests are used to show the presence of antibodies against corpuscular antigens, through the agglomeration of antigen/antibody complexes which causes the formation of a precipitate (referred to as "agglutinate" in the art) visible to the naked eye. In other words, the term "agglutination" indicates a phenomenon through which, under specific conditions, bacteria, red blood cells, and other corpuscular elements suspended in a liquid assemble in clumps and precipitate, and it is thus the visible manifestation of the aggregation of antibodies and antigens.

The above-mentioned serum-agglutination tests allow to verify the positivity of a sample and also the semiquantitative determination of antibodies through titration, which is an operation consisting in the use of progressive dilutions of the serum under test and detection of the highest dilution (i.e. lowest concentration) at which the antigen/antibody reaction causes agglutination ("antibody titer").

The agglutination tests are simple to perform and have a wide range of applications to infectious and non-infectious diseases. For example, it is possible to make a diagnosis of enteric fever/typhus fever through bacterial agglutination (in which the antigen - hereinafter referred to as "Ag" - consists for example in a bacterial suspension), or a diagnosis of syphilis through an RPR test (Ag with charcoal) and/or TPHA test (Ag on Red Blood Cells), as well as rheumatology tests (for example, ASO, RF, CRP with Ag on latex), diagnosis of mononucleosis (Ag on latex) and determination of the ABO blood group.

To this day, said methods are still the reference test for the diagnosis of some infectious diseases such as for example salmonellosis, brucellosis, and syphilis, and are often performed manually (for example, by performing doubling dilutions of the serum under test) with a variety of approaches (on a slide, on suitable cardboards, in a tube, in a microplate). Manual tests require a visual interpretation of the data and the results are often obtained after long incubations (lasting hours) of the reaction mixture, wherein the analysis is hardly objective and it is difficult to interpret the result, since the interpretation is highly dependent on the operator's ability and experience.

There are apparatuses capable of performing serum-agglutination tests in an automatic way, for example through the analysis of the images of the microplate containing the samples, although said apparatuses are often not free from flaws, such as for example the difficulty of extracting from the image analysis features that are not the mere positive/negative discrimination, as well as their poor reliability.

The technical problem of the present invention is to provide an apparatus having functional and structural features capable of overcoming the limitations and the drawbacks reported in connection to the prior art, in particular an apparatus that allows to perform serum-agglutination tests in an automatic and reliable way.

### Summary of the invention

The solution idea underlying the present invention is to make an apparatus capable of performing in an automatic way serum-agglutination tests through automated analysis of images of the microplate containing the reaction wells; said analysis allows to extract numerous significant features from the acquired images, thereby obtaining highly deterministic metric features in relation to the sample displayed in said images, such as for example blob detection, the detection of closed contours and the identification of specific form factors (circles, ellipses or portions thereof), thereby obtaining a reliable analysis thanks to a combination of said features.

Based on said solution idea, the above-mentioned technical problem is solved by an apparatus for performing serum-agglutination tests, comprising a housing area configured to house a plate with a plurality of wells containing a sample to be analyzed, a processing unit adapted to manage the apparatus, and at least one image detector in communication with the processing unit for acquiring images of the plate located in the housing area (partial or whole images thereof), wherein the processing unit comprises instructions for processing the acquired images which, when executed, cause it to carry out the following steps (i.e., the processing unit is configured/programmed to): controlling the acquisition of at least one image containing at least one well (for example, from the image of the plate, the single wells may be cut out and individually analyzed), extracting a plurality of features from the image, said features comprising at least one number of aggregates of pixels detectable in said image, a number of closed contours detectable in said image, and a number of elements having a determined at least partially circular and/or elliptical geometry detectable in said image (closed shapes, portions thereof, and also arcs) and classifying, based on a combination of said features, the sample contained in the well, providing information on the positivity of said sample through said classification.

More in particular, the invention comprises the following additional and optional features, taken individually or in combination if needed. Said aspects are in particular described, for example, in the dependent claims 2 to 15, individually or in combination with each other.

According to an aspect of the present invention, the processing unit may be configured to assign a class to the sample contained in the well, the class being selected from at least one first class of negative samples, wherein a formation of a substantially uniform ring is detectable in the image, one second class of positive samples, wherein in the image there is absence of the ring or an incomplete ring, and at least one third class intermediate between said first class and said second class (uncertain class).

According to an aspect of the present invention, the processing unit may be configured to identify the number of elements having a determined circular geometry in the image by executing a calculation procedure based on the Hough transform for searching circles.

According to an aspect of the present invention, the processing unit may be configured to identify the number of elements having a determined elliptical geometry in the image by executing a calculation procedure based on a least squares minimization for searching ellipses.

In these cases, the above-mentioned procedures are also capable of identifying portions of circles/ellipses or arcs.

According to an aspect of the present invention, the aggregates of pixels detectable in the image (namely, the blobs) may be defined as a set of contiguous dark (e.g. black) pixels with an overall area beyond (e.g. smaller than) a predetermined threshold.

According to an aspect of the present invention, the processing unit may be configured to calculate the number of closed contours detectable in the image having a perimeter (or other equivalent magnitude) beyond (e.g. larger than) a determined threshold.

According to an aspect of the present invention, the processing unit may be programmed to identify a closed contour, such as circular, based on a pre-fixed relation between area and perimeter of said closed contour.

According to an aspect of the present invention, the processing unit may be configured to to identify, inside the plate, single wells and to cut the image so as to process said image. For example, the identification of the single ones may be performed by identifying lines corresponding to the separation lines of the strips that form said plate.

According to an aspect of the present invention, the processing unit may be programmed for associating with the identified single wells respective samples whose features (for example, the name or other information about the patient) are defined in a memory unit thereof.

According to an aspect of the present invention, the processing unit may be configured to binarize the image (in particular the image of the single well) prior to extracting the features

According to an aspect of the present invention, the processing unit may be configured to obtain (e.g., by cutting the image of the plate) and process a plurality of images of a respective plurality of wells, each of said wells corresponding to a specific serial dilution of the sample therein contained, thus producing as output a titration curve in relation to the sample contained in said wells. For each dilution, the processing unit is configured to perform the classification of the sample.

According to an aspect of the present invention, the processing unit may be programmed to automatically identify, inside the plate, based on the instructions therein stored, a series (array) of said wells, each of which with serially diluted sample, and analyze said wells consecutively to obtain the above-mentioned titration curve. This curve may then be compared with stored curves.

According to an aspect of the present invention, the processing unit may be configured to perform the classification of the sample by executing a tree-decision algorithm, which is structured to define, for each of the extracted features, optimal threshold values in order to classify the sample, said tree-decision algorithm being trained based on example images containing formations of antigen/antibody agglomerates relating to a status of a patient.

According to an aspect of the present invention, the processing unit may be configured to define, based on the combination of said features extracted from the image, a percentage of positivity of the sample.

According to an aspect of the present invention, the processing unit may be configured to process the images acquired by means of machine-learning techniques.

According to an aspect of the present invention, based on an acquired image (for example, a preliminary image, but non necessarily a preliminary image, it may also be the image that is then processed as described above), the processing unit may be configured to verify the actual presence of the plate in the housing area, to verify that said plate is the plate associated with a set test, and to verify the orientation of the plate relative to a reference system.

According to an aspect of the present invention, the apparatus may comprise a door which acts as a closing element of the housing area and which is configured to pass from an open configuration, in which it is possible for the operator to access the housing area, to a closed configuration, in which it is not possible to access said housing area and vice versa, said door being provided with a solenoid configured to allow a magnetic closure thereof during the test.

According to an aspect of the present invention, the processing unit may be programmed to perform, based on said image processing, a test of the Rapid Plasma Reagin (RPR) type, using an antigen containing (formed of) at least cardiolipin, possibly supplemented with lecithin and cholesterol, and possibly mixed with particles for a visual contrast (such as, for example, charcoal) for detecting reagin.

According to an aspect of the present invention, the processing unit may also be programmed to perform tests of the Treponema Pallidum Haemagglutination Assay (TPHA), Widal Wright (WW) and Weil Felix (WF) type.

The present invention also relates to a method for performing serum-agglutination tests, comprising the steps of:
- controlling the acquisition of at least one image containing at least one reaction well of a plate;
- extracting a plurality of features from the image, said features comprising at least one number of aggregates of pixels detectable in said image, a number of closed contours detectable in said image, and a number of elements having a determined at least partially circular and/or elliptical geometry detectable in said image; and
- classifying, based on a combination of said features, the sample contained in the well, providing information on the positivity of said sample through said classification.

The features and advantages of the apparatus and the method according to the invention will become apparent from the following description of an embodiment thereof, given by way of non-limiting example with reference to the accompanying drawings.

### Brief description of the figures

In the figures:
- Figure 1 is a view of the apparatus according to the present invention;
- Figure 2 shows an intentionally simplified outline of the apparatus according to the present invention;
- Figure 3 is an exemplary image of a plate containing a plurality o reaction wells;

- Figure 4 shows an image of a plate portion in which with each well is associated a respective sample;
- Figures 5A-5D show examples of images of a reaction well for a negative, an uncertain, a weakly positive and a strongly positive sample in the case of an RPR test;
- Figures 6A-6D show examples of image processing according to the present invention;
- Figure 7 shows a table with examples of features extracted from well images of an RPR test;
- Figure 8 is an example of definition of percentage of positivity of a sample in an RPR test;
- Figures 9A-9D show examples of images in the case of a TPHA test; and
- Figures 10A-10D show examples of images in the case of WW and WF tests.

### Detailed description

With reference to those figures, an apparatus for performing serum-agglutination tests according to the present invention is globally and schematically indicated with the reference number 1.

It is worth noting that the figures represent schematic views and are not necessarily drawn to scale, but instead they are drawn so as to emphasize the important features of the invention. Further, in the figures, the different elements are shown in a schematic way, their shape being variable depending on the desired application. It is further worth noting that in the figures identical reference numerals refer to identical elements in shape or function. Finally, particular features described in relation to an embodiment illustrated in a figure are also applicable to other embodiments illustrated in the other figures.

It is also noted that, unless the opposite is expressly indicated, the described process steps may also be inverted if necessary.

The present description shows an apparatus applicable to the diagnosis of infectious diseases such as syphilis, even if the present invention is not limited to this application.

In particular, the following detailed procedure is applied to performing RPR (an acronym for "Rapid Plasma Reagin") tests, i.e. a non-treponemal test based on the detection of reagins, a class of antibodies found in syphilis and sometimes also in other acute and chronic pathologies. As an antigen, a purified extract of beef heart (cardiolipin), possibly supplemented with lecithin and cholesterol, may be used, substantially providing a screening characterized by practicality and reproducibility.

In other words, it is an analysis of non-treponemal agglutination reactions for searching aspecific antibodies against T. Pallidum, for example for searching IgG/IgM antibodies specific for the lipidic antigens released by the damaged cells of the host and/or specific for the cardiolipin released by the Treponema.

In an embodiment, the RPR test uses a reagent containing particles (in particular, microparticles) to create a visual contrast, such as, for example, charcoal.

The apparatus 1 may be programmed to also perform, in addition to the above-mentioned test, other types of analysis, even if the present description and the following examples will regard the RPR test. As known, in the case of an RPR test, due to the presence of the particles for visual contrast, after the sample has been combined with the reagent, the formation of a ring is obtained in the case of negative samples, whereas there is absence of a ring or presence of an incomplete ring in the case of positive samples, and the apparatus 1 is capable of verifying it, as will be detailed hereinafter.

For the purpose of allowing the execution of the operations described below, the apparatus 1 comprises a processing unit (identified with reference C), including an appropriate memory unit MEM and suitably programmed and designated for managing and automatically controlling the apparatus and for the analysis of the data of measurement. The processing unit C may be, for example, a computerized unit integrated in the apparatus 1. Moreover, it is noted that the processing unit C may be a single unit or may comprise a plurality of local and/or remote units, possibly communicating with each other and each being designated for performing specific operations. The processing unit C is thus capable of controlling the apparatus 1 to obtain the desired functionalities. In any case, the present invention is not, in any way, limited by the architecture used for the processing unit C, which may be in general any suitable computerized unit, comprising one or more unit(s) depending on the needs and/or circumstances. Only by way of example, an electronic board IMX8 may be used as CPU.

In any case, regardless of the hardware configuration used, the processing unit C is programmed to verify that, when the sample is combined for example with a colloidal suspension of cardiolipin, lecithin, and cholesterol mixed with microparticles for visual contrast (such as, for example, charcoal), there is or there is not the presence of a ring in the reaction well, as will be detailed below.

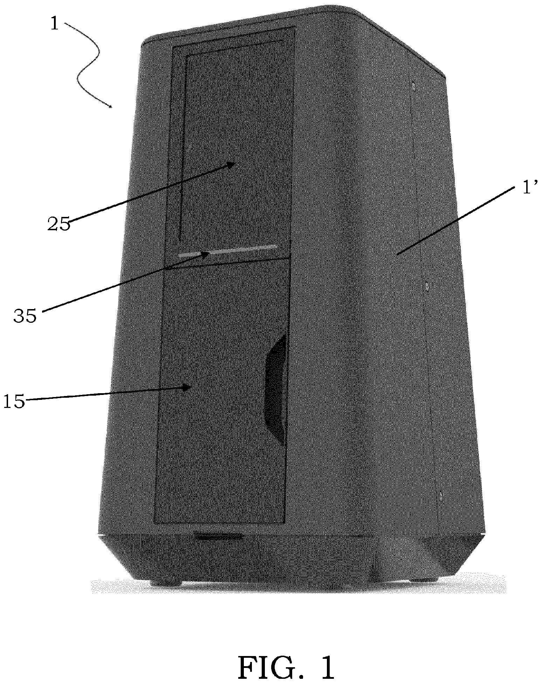

As illustrated in Figure 1, in its most general form, the apparatus 1 comprises a casing (reference 1') containing its main hardware components, but it is not limited to a given aesthetic shape.

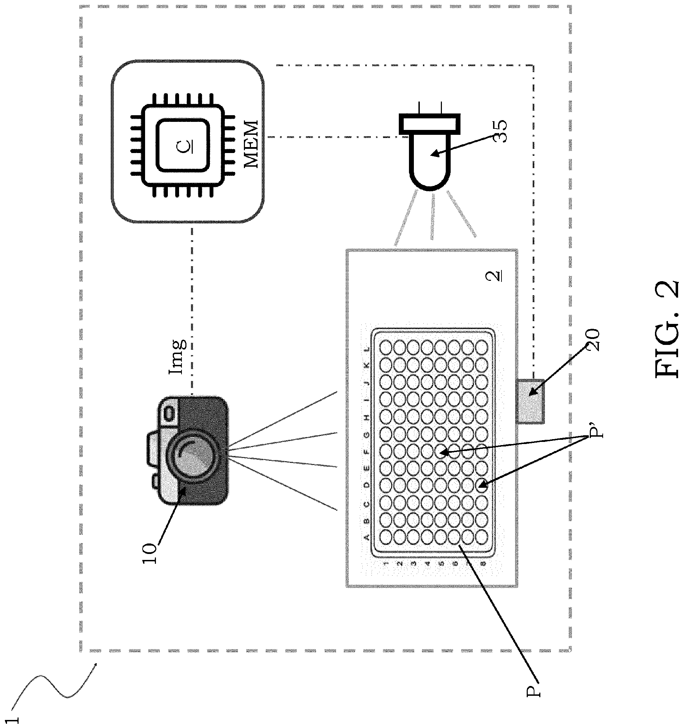

As schematized in Figure 2 (which is an intentionally schematic illustration of the instrument), there is also a housing area (reference number 2) configured to house a plate (or microplate, indicated with the reference P) containing a plurality of reaction wells P' in which there is a sample to be analyzed. By way of example, the plate P may contain ninety-six reaction wells.

The apparatus 1 further comprises a reading unit including at least one image detector (reference 10) in communication with the processing unit C for acquiring images (indicated with the reference Img) of the plate P arranged in the housing area.

In an embodiment, the image detector 10 is a camera having high resolution (for example, from 10 MP to 20 MP) and capable of adjusting the focus, the exposure and other parameters. The camera may also be provided with ad hoc optics capable of minimizing the distortion effect. Moreover, there may be two cameras (each camera framing a respective plate portion and the overall image being thus given by the combination of the images of the two cameras), however, the present invention is not limited by said configuration.

The processing unit C is configured to process the images Img acquired by the image detector 10 and to perform, based on said processing (in particular, based on an image-processing algorithm), a serum-agglutination test relative to the samples contained in the wells P' of the plate P. The purpose of said processing is to extract a plurality of features (defined as "features" or "parameters" and generally indicated with the reference F) from the image Img, as will be detailed hereinafter.

The following description will detail the procedure of image processing for performing RPR tests.

First of all, a preliminary procedure of verification of the correct setup of the apparatus 1 is performed. For example, in this preliminary procedure, an image Img of the plate P is acquired and, based on this image Img, it is possible to verify the actual presence of the plate, P, and it is also possible to verify that said plate P is exactly the plate associated with the set test, i.e., if it is the correct plate for said test. As it will be discussed hereinafter, the apparatus 1 is in fact capable of performing different agglutination tests, each characterized by its own specific plate, and thus allows the user to set the desired test, said setting corresponding to the automatic selection, by the processing unit C, of the respective operating instructions, which is the reason why it is important to verify that the plate P inserted is the correct one. In this preliminary procedure, it is further possible to verify the orientation of the plate P according to a pre-fixed reference system.

These preliminary procedures, as well as other processing steps discussed below, may be performed by means of machine-learning techniques.

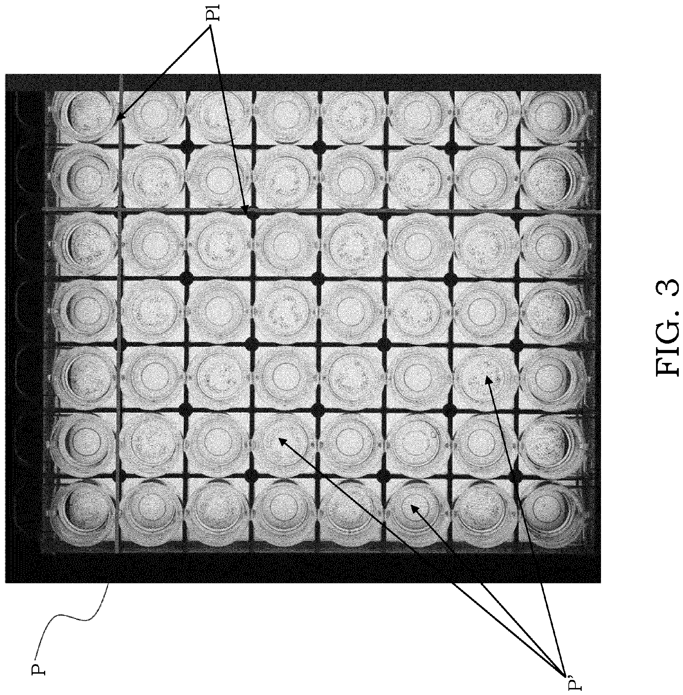

For what concerns the specific procedure of image processing for performing the test, first of all, the processing unit C cuts out the regions of interest to be processed. A first layer of the algorithm thus deals with cutting out each well P' of the plate P, identifying the lines (reference Pl) corresponding to the separation of the various strips of which the plate P is formed, for example using the HoughLine library of openCV, without, however, that the present invention is limited to this specific library. Only by way of example, Figure 3 highlights two of the above-mentioned lines Pl, which correspond to dark lines. The so-found lines P1 constitute thus a grid and each rectangle defined by said lines P1 delimits a single well P' whose center coincides in this way with the center of the rectangle. Once defined the grid as described above, the distribution of the distances of the horizontal lines and the distribution of the distances of the vertical lines are calculated. Then, the median values of said distributions are identified, which are subsequently used to exclude possible outlier lines (i.e., not corresponding to an actual separation between the strips of the plate P) and to reduce the effects of perspective distortion of the image detector 10. Subsequently, in an embodiment, for each rectangle, the well P' is identified by cutting out a circular area of 120-pixel radius with reference the rectangle center.

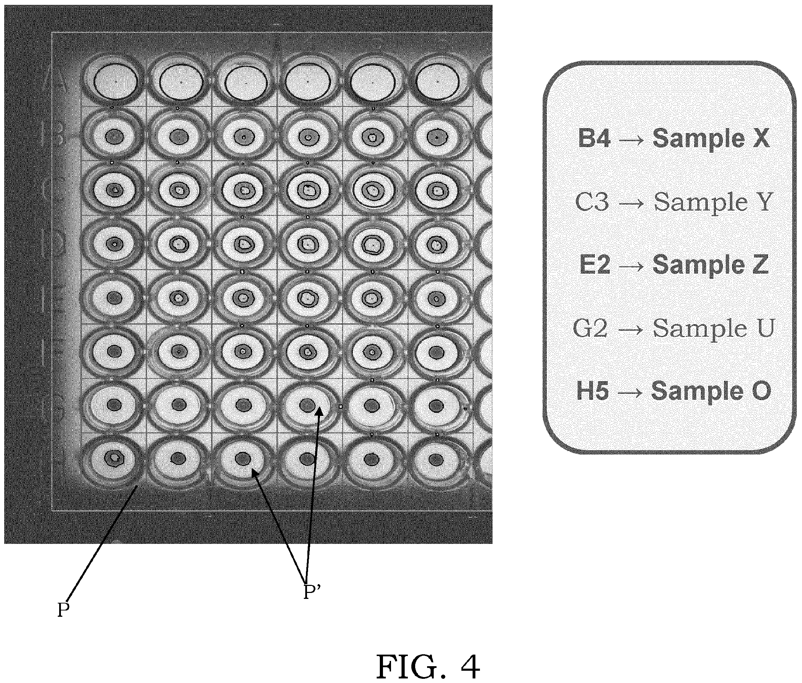

It is also noted that, since the plate P may contain a high number of wells (for example, ninety-six), the processing unit C is programmed to associate with the identified single wells P' respective samples whose features (for example, the data identifying the patient) are defined in its memory unit MEM, as shown in Figure 4 (showing the image of a portion of the plate P), in which with each position of the well in a defined grid is associated the respective sample.

In brief, according to the present invention, the processing unit C is configured to identify, inside the plate P, single wells P' by identifying the lines Pl corresponding to the separation lines of the strips that form said plate P, and subsequently the processing of the images of the single wells P' is performed. For the sake of easier description, the images of the single wells P' will be always indicated with the reference "Img" used for the image of the plate P.

Suitably, the processing unit C is further configured to binarize the acquired image Img, in particular the image of the single well P', prior to extracting the features F of interest, so that said features F are obtained from the binarized image Img.

For example, the image of the single well P' is first translated into gray scale and subsequently binarized. More in particular, three different binary images are produced, calculated based on three different adaptive approaches of threshold extraction, i.e. an image by means of Otsu binarization, an image by means of K-means algorithm, and an image by means of Otsu binarization with reduced thresholding.

For each of said three binarized images, an image is further created by means of an erosion algorithm with 3x3 Kernel and an image by means of an erosion algorithm with 7x7 Kernel; this operation of erosion of the binarized image allows to obtain a closed circle even in the case in which the reaction has discontinuous circles or circles having nonuniform intensity. In general, the binarization and possibly the erosion allow to improve the contrast to make the identification of the features F easier, in particular to identify the circle (in particular, as closed as possible).

In this way, for each well P', a series of nine binarized images is defined, on which images, some quantities characterizing the image Img are then extracted, as described below.

In particular, advantageously according to the present invention, as mentioned above, the processing unit C is configured to extract a plurality of features F from the image Img of the single well P'. More in particular, for each image Img the following features F are calculated.

The perimeter and the area of the blobs that form following agglutination are calculated, in particular, the number of blobs having a perimeter and an area in relation to a chosen threshold (said parameters being referred to as "BlobLenght" and "BlobArea", respectively) is determined, wherein the term "blob" indicates any set of contiguous black pixels with an area smaller than a predetermined threshold. In an embodiment, in this determination, only the images that are not processed by the erosion algorithms and are obtained by means of the binarization processes by means of the K-means and Otsu methods are used, extracting the average value of said two binarization configuration.

In other words, the configuration unit C is programmed to determine a number of aggregates of pixels detectable in said image Img, said aggregates of pixels being defined as a set of contiguous black pixels with an overall area smaller than a predetermined threshold.

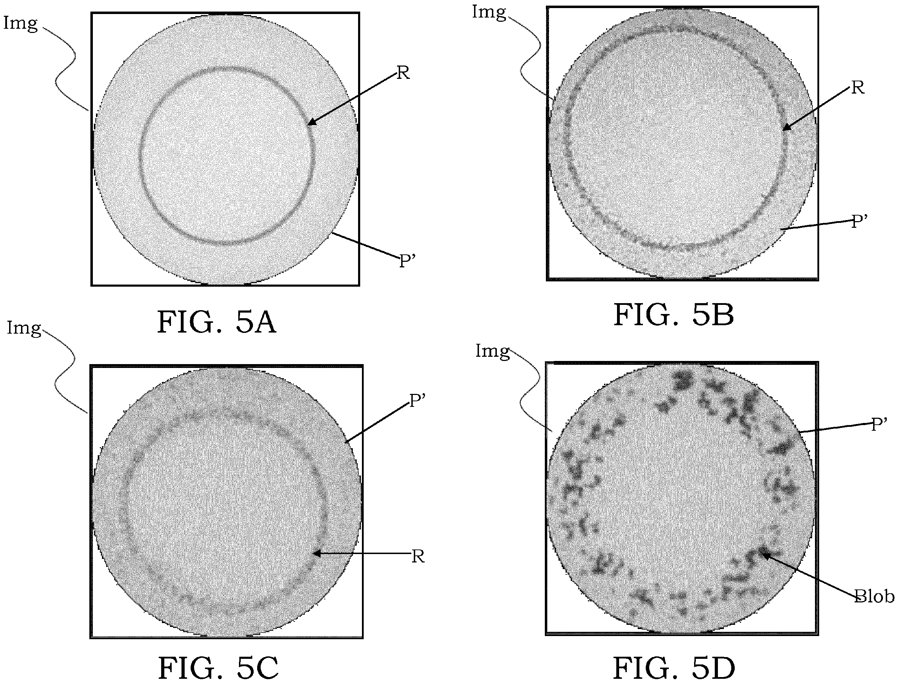

This determination is significant since, in RPR tests, after the sample has been combined with the reagent, in the well P' there is the formation of a ring (indicated with the reference R) which is well defined in the case of a negative sample, whereas there is absence of a ring or presence of an incomplete ring in the case of a positive sample, and for this reason it is important to identify within the image of the well possible clumps of particles or fragments in addition to the rings to ascertain the possible positivity of the sample. Figures 5A-5D show images of wells for a negative, an uncertain, a weakly positive and a strongly positive sample, respectively, the images being obtained by means of the apparatus 1 of the invention and the classification being performed in the way herein described above.

The number of closed circular contours identified in the image Img having perimeter above a fixed threshold is determined. From this measurement it is then possible to define a parameter (referred to as "Contours" below) as the sum of the circular contours found in all the image configurations obtained (for example, in all the binarized and erosion-subjected images). For example, a contour is defined as circular when a parameter calculated as 4 * π * area/ (perimeter²) is greater than 0.5, without however being a limiting example of the scope of the present invention.

In other words, the processing unit C is configured to calculate the number of closed contours detectable in the image Img that have a perimeter above a determined threshold, wherein a closed contour is defined as circular based on a pre-fixed relation between area and perimeter of said closed contour, as discussed above.

Finally, by means of shape-recognition algorithms for the identification of circles and ellipses, a number of elements having, at least partially, circular and/or elliptical shape is identified and said number is registered. In an embodiment of the present invention, the number of elements having a determined circular geometry in the image Img is obtained by means of an algorithm based on the Hough transform ("HoughCircle" algorithm) for searching circles, whereas the number of elements having a determined elliptical geometry in the image Img is obtained by means of an algorithm based on a least squares minimization for searching ellipses (fitEllipse algorithm). More in particular, the algorithm HoughCircle is based on the implementation of a mathematical technique known as Hough transform, applied in this case to the circular curves, wherein searching a circular shape is made by searching, for each point of the image Img, a conical surface in the 3D space of the parameters center (in the x and y coordinates in the plane of the image) and radius; said surfaces, if indeed originating from the same circle, will have intersection points in the space (also called accumulation points).

A global parameter (here called "HoughCircle") is defined as the sum of the number of candidate circles produced through the HoughCircle library and of the number of ellipses in the various binarization configurations previously described.

In the latter analysis, it is also provided the detection of the number of arcs from the image Img, and the parameter "HoughCircle" thus considers both whole circles (or also ellipses) and possibly arcs.

For each binary image are thus measured the above features and the output of the test is obtained based on the amount of extracted features in all the configurations and on their combination. For example, the positive samples do not have closed contours (i.e., the parameter "Contour" is zero or very low) and have a low number of circles/arcs ("HoughCircle").

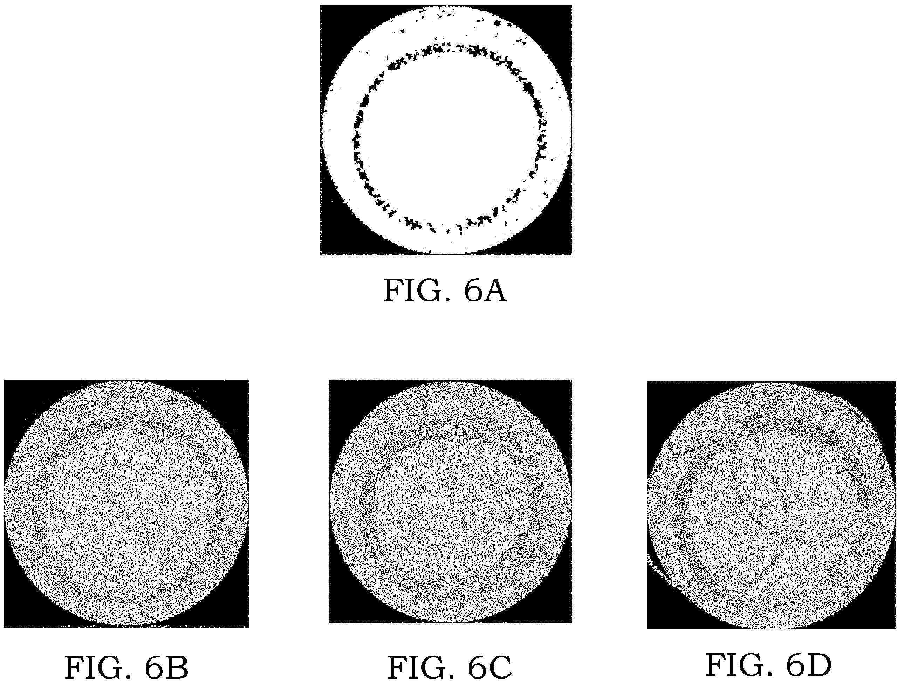

Figures 6A-6D show examples of processing of the acquired image, wherein Figure 6A is an example of binarized image and Figures 6B-6D are examples of processing of the structures formed by means of specific procedures of searching shapes.

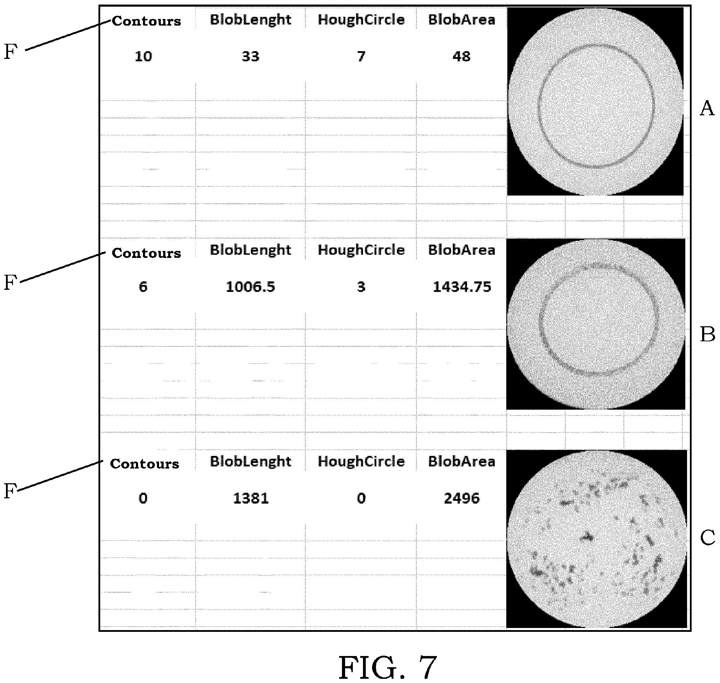

Figure 7 shows the above-mentioned extracted parameters "Contours", "BlobLenght", "HoughCircle" and "BlobArea" for the images relating to a negative (row A), uncertain (row b) and positive (row C) sample, which globally define the metric of the sample depicted in said images, in particular the metric of the positivity level.

In other words, summing up the above, according to the present invention, the features F comprise at least one number of aggregates of pixels detectable in said image Img (blob detection, in particular the above-mentioned parameters "BlobLenght" and "BlobArea"), a number of closed contours detectable in said image Img (the parameter "Contours"), and a number of elements having a determined at least partially circular and/or elliptical geometry detectable in said image Img (the parameter "HoughCircle").

Once the extraction of the above-mentioned features F has been performed, it is then possible to classify, based on a combination thereof, the sample contained in the well P', providing information on the positivity of said sample through said classification.

In particular, in an embodiment of the present invention, the processing unit C is configured to assign to the sample contained in the well P' one of the following classes:
- a first class of negative samples, wherein the formation of a substantially uniform ring (as in Figure 5A) is detectable in the image Img of the respective well P';
- a second class of positive samples, wherein in the image Img there is absence of the ring or an incomplete ring (as in Figures 5C and 5D); and
- at least one third class intermediate between said first class and said second class, indicated below as uncertain class (as in Figure 5B).

Said accurate classification is possible thanks to the many deterministic features extracted from the image Img, which fact allows a high analysis resolution, which is not based on the mere search for the presence or absence of rings in the well but provides the extraction of precise metric features from the image.

In an embodiment of the present invention, the above-mentioned classification is performed by means of a tree-decision algorithm, which is structured to define, for each of the extracted features F, optimal threshold values in order to classify the sample. The tree-decision algorithm is trained based on example images representative of known samples, for example containing formations of antigen/antibody agglomerates relating to a status of a patient. By way of example, for this algorithm, Python libraries sklearn may be used. Specifically, the tree-decision algorithm is structured to find the optimal threshold values of the above-mentioned features "Contours", "BlobLenght", "HoughCircle" and "BlobArea" in order to classify the sample into the three classes through binary decisions on the values of said parameters. Specifically, for each feature, the tree-decision algorithm defines a threshold value under which the purity of the sub-samples (in terms of classes contained) is maximum. The algorithm may be set so as to determine in advance the number of leaves of the tree (said number being directly related to the maximum number of binary conditions that it is authorized to use in order to optimize the accuracy of the prediction and the purity of the samples); only by way of example, it is possible to use a value of 7. This algorithm may be trained from standard training libraries sklearn, providing for each image a classification created by a biologist specialized in the pathology.

In any case, it should be noted that the determination of the class to which each sample belongs is not necessarily obtained by using said tree-decision algorithm, and the thresholds may also be defined in advance by the operator based on preliminary studies on known images. In any case, a threshold is set for each of the measured parameters and the combination of the results for each of said parameters allows to obtain the desired classification, said threshold being possibly adjustable.

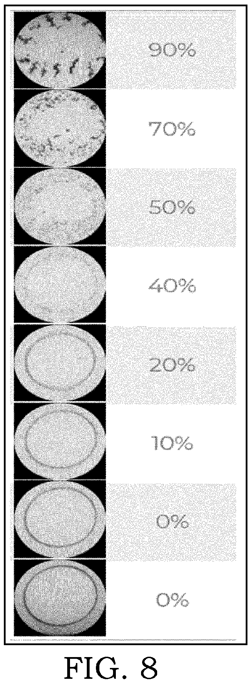

Moreover, according to a non-limiting aspect of the present invention, the processing unit C is configured to define, based on the combination of the features F extracted from the image Img, a percentage of positivity of the sample (for example, in the case of the RPR test, the maximum percentage corresponds to the maximum positivity degree, whereas the minimum percentage corresponds to a well-defined ring and thus to a negative sample, as illustrated in Figure 8). In this case, it is possible to set a positivity threshold from this percentage value, i.e., it is possible to define a percentage value beyond which the sample is considered positive, thereby obtaining an even finer analysis.

In an advantageous embodiment of the present invention, a plurality of images of a respective plurality of wells (for example, wells that are adjacent in the microplate) is acquired, each of said wells corresponding to a specific serial dilution of the sample therein contained; thereby, it is possible to produce as output a titration curve in relation to the sample contained in said wells P, in which, for each dilution, a classification of the sample is performed. Essentially, in these wells P', the same amount of antigen is used with progressive dilutions of the serum under test, and the processing unit C is capable of detecting which is the highest dilution at which the antigen/antibody reaction causes agglutination (thereby providing the so-called "antibody titer"). Said titration curve may then be compared with reference titration curves stored in the memory unit MEM.

In the case in which the percentage of positivity of the sample is also defined, a quantitative result is obtained in a single well, thus allowing to perform a smaller number dilutions of the sample (thereby saving space in the microplate).

Suitably, the processing unit C is programmed to automatically identify, inside the plate P and based on the instructions therein stored, the series of said wells P' with serial dilution and to analyze said wells in a consecutive way, i.e., it is capable to analyze, by means of prearranged instructions, arrays of wells with serial dilutions.

Generally, the above-described processing procedure is particularly advantageous since it allows to extract deterministic features from the image, with higher precision on the determination of the thresholds to classify the samples, and thus with a higher reliability of the measurement and also the possibility to obtain a semiquantitative analysis.

The operation of the specific program of processing the images Img acquired through the apparatus 1 for performing the RPR test was described above. As regards the general operation of the apparatus 1, instead, the measurement cycle comprises various steps, and the programming of a cycle may occur both for a single test and for diagnostic profiles, which is particularly useful in the case of bacterial-agglutination tests in which a single serum is tested with different bacterial suspensions. The reagents and the samples are dispensed manually into the plate P following precise indications and, once inserted in the housing area 2 of the instrument, said plate P is processed as follows:
- rapid shaking to mix the sample with the reagent;
- image acquisition at time zero;
- incubation under rotation at constant speed for the time predefined by the specific method (the reaction times are preset and depend on the specific test);
- image acquisition;
- image processing (for example, in the way described above); and
- reading and printing the test results.

As indicated above, the apparatus 1 is configured for the analysis of non-treponemal agglutination reactions for searching aspecific antibodies against T. Pallidum, i.e., RPR tests, in which the images are analyzed with the above method. A non-treponemal test for serological screening of syphilis is then performed, and the use of microtiter plates of the breakable type is provided. As discussed above, after the sample has been combined with the reagent (for example, antigen of cardiolipin, lecithin, and cholesterol mixed with microparticles for visual contrast - such as, for example, charcoal), in the well P' there is the formation of a ring (for example, a charcoal ring) in the case of negative samples and there is absence of a ring or presence of an incomplete ring in the case of positive samples, and said analysis allows to analyze in detail this feature.

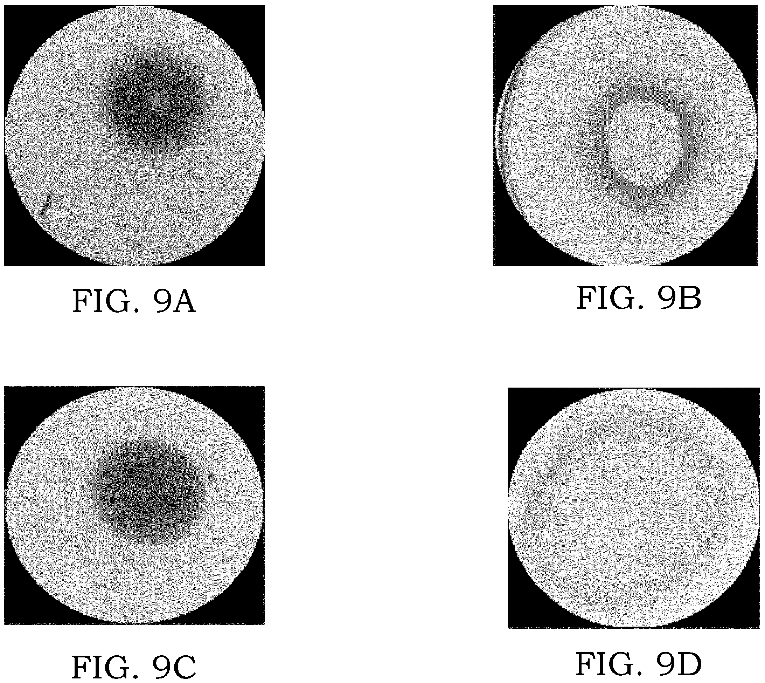

In addition to said test, the apparatus 1 is capable of performing also other serological tests, such as the analysis of reactions of treponemal hemagglutination for searching T. pallidum-specific antibodies, i.e., the TPHA test (acronym for Treponema Pallidum Haemagglutination Assay), wherein specific antibodies in the serum of the patient bind to the treponemal antigens attached to avian erythrocytes, thereby allowing to confirm the positivity to syphilis. In this case, U-bottom microtitration plates may be used. Figures 9A-9D show examples of images obtained and analyzed in a TPHA test. In this case, the processing unit C is configured to verify that, when the sample is combined with treponemal antigens attached to avian erythrocytes, in the image Img a dispersed agglomerate characterized by a circle having a certain perimeter and/or the presence of a ring defined in the agglutinate is formed.

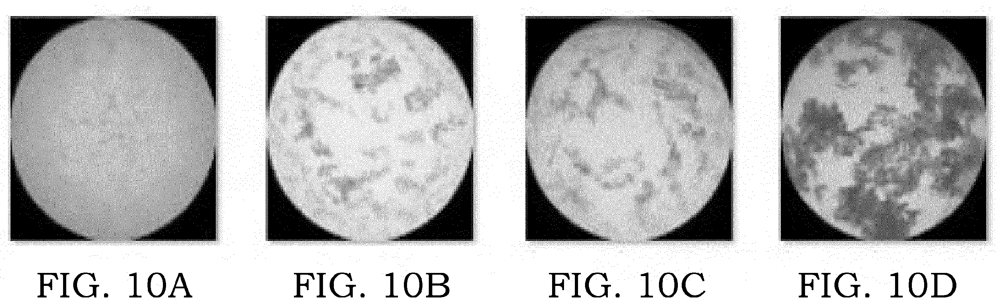

Moreover, the apparatus 1 is capable of analyzing bacterial-agglutination reactions for the detection of antibodies against Salmonella, Brucella and Rickettsia, namely, it is also capable of performing the Widal-Wright (WW) and the Weil-Felix (WF) tests. In this case, seven specific bacterial suspensions (possibly colored) may be used for the serological diagnosis of infections by Salmonella and Brucella (TO, TH, AO, AH, BO, BH, BR) together with a positive-control polyvalent serum, and three specific bacterial suspensions (possibly colored) for the serological diagnosis of infections by Rickettsia (Proteus OX19, OXK, OX2) together with a positive-control polyvalent serum. Figures 10A-10D show examples of images obtained and analyzed in WW and WF tests. In this case, the processing unit C is configured to verify whether or not there is any agglutinate in the image Img when the sample is combined with a specific bacterial suspension (possibly colored).

The following paragraphs regard details of the mechanical structure of apparatus 1.

As seen above, there is the casing 1 containing all the main components of the apparatus 1, among which the above-mentioned housing area 2. In connection with the latter, it has the function of receiving and holding the plate P prepared by the operator steady inside the instrument, and it acts as the direct interface between the machine and the element to be analyzed. The housing area 2 is slightly flexible to enable to insert easily the microplate inside it, but it is also sufficiently rigid to be hold steady during the steps of shaking and rotation. The housing area 2 is compatible with microplates of the Greiner BioOne 96 stipwells breakable type (for performing the RPR test, and said plate allows to perform the above-mentioned procedure of cutting out the single well from the image of the plate P through identification of the lines Pl), and it is also compatible with microplates of the BioLab 96 well microplate type for performing other tests.

In an embodiment of the present invention, the apparatus 1 comprises a door (reference 15) which acts as a closing element of the housing area 2 and which is configured to pass from an open configuration, in which it is possible for the operator to access the housing area 2 to be able to insert or remove the plate P, to a closed configuration, in which it is not possible to access said housing area 2, and vice versa. For security reasons, the door 15 may be provided with a solenoid configured to allow a magnetic closure thereof during the test. The closure of the door 15 may be managed by means of designated software commands inputted through GUI. In an embodiment, there is also a sensor on the door 15 to allow the control of the correct closure thereof, for example a microswitch.

The images Img acquired through the apparatus 1 are saved on site in the memory unit MEM together with the analysis results and are thus available for possible visual checks; the data of measurement, including the instrument's settings, are contained in a designated database.

The apparatus 1 may also connect in a bidirectional way with the Laboratory Information System (LIS), for a complete traceability and security of the analytical data.

As regards the orbital shaking of the microplate, the apparatus 1 further comprises a shaker (reference 20) which is adjustable in speed through appropriate software commands. The shaker 20 thus moves the microplate (for example, it moves said housing area 2 or a support therein) and, in an embodiment, it comprises a step motor which, using helicoidal gears, allows to perform two types of movements for carrying out the steps of analysis, namely, a slow rotation and a fast rotation.

Moreover, the apparatus 1 comprises a display (reference 25), for example a touch-screen display, which may be tilted to allow an easier interaction with the operator. In addition to the operating settings, on the display 25 it is possible to display the acquired images and to confirm the positivity of a sample.

There is also a reader for the identification of the barcode of the samples and of the reagents, and an internal temperature sensor for measuring the inner temperature for the apparatus 1 during the analysis cycle may also be provided.

Moreover, the apparatus 1 comprises a lighting system (reference 30) to illuminate the working area. In particular, there is a first internal lighting system arranged above the plate P which allows to check the lot and the orientation of said plate P with the door 15 in closed configuration. Moreover, under the housing of the microplate, there is a second lighting system (for example, a LED lighting board) for backlighting the wells P'.

Furthermore, on the casing 1', there is a luminous indicator (reference 35, for example an RGB LED) which allows to indicate to the user the current working status of the apparatus 1.

In the light of the above, it is clear that the present invention also refers to a related method for performing serum-agglutination tests, in particular for performing RPR tests.

In conclusion, summing up, the present invention allows to overcome the technical problem brilliantly, providing the above apparatus and solving all the drawbacks of the prior art, wherein, when performing an RPR test, numerous features of the image are extracted through computer-vision techniques, thereby possibly allowing to also provide a semiquantitative result and an antibody titer.

It is thus possible to perform serum-agglutination tests in microplate in an automatic and efficient way, obtaining up to ninety-six results in 15/25 minutes and providing a semiquantitative output in a single well, with archiving of results and images and a complete traceability of the results.

The apparatus 1 is very compact and is capable of carrying out the steps of mixing, incubation and reading in an automatic and efficient way, and the operator is only required to prepare the microplate.

Reading and objective interpretation of the reactions are obtained thanks to the innovative image-processing program described above. Suitably, the image processing is extremely detailed since it allows to extract a very precise metric of the sample, with a higher reliability of the test results. The extraction of a high number of visual features thus enables an effective sample classification. In this way, it is possible to make a precise identification of negative samples even in the cases in which the circle did not form in a perfectly circular way, thanks to the performed analysis and to the high number of features compared, which are not limited to the mere identification of the ring. In fact, no direct analysis on the histogram of the images is made, but the images are first binarized and then the various features are extracted. Moreover, the analysis is very precise even in the case in which the reaction does not occur in the center of the well (for example, due mechanical problems). Moreover, there is an output of "uncertain" class, which may occur due to the non uniformity of quality of the solutions, presence of bubbles, or degradation of the instrumental mechanics.

Summing up, the present invention allows obtaining a very precise and accurate classification thanks to the several deterministic features extracted from the image, with a very high analysis resolution which is not based on the simple search for the presence or absence of the rings. Advantageously, the processing unit configured to extract various precise metric (geometric) features (such as for example a combination of search for close contours and elements having a circular and/or elliptic geometry and possibly aggregates of pixels), so as obtain a fine and precise metric from the image in order to perform the classification of the sample, including the possibility of defining also at least one third class that is intermediate between the first class and the second class (i.e., the class relating to dubious/not clear sample output). The definition of said third class is very important, and it occurs for example in case of bad quality or nonuniformity of the samples, presence of bubbles, or also problems in the apparatus mechanics.

Furthermore, the present invention allows defining in an useful way the region of interest.

Obviously, a person skilled in the art, in order to meet particular needs and specifications, may carry out several changes and modifications to the apparatus described above, all included in the protection scope of the invention as defined by the following claims.

## Claims

1. An apparatus (1) for performing serum-agglutination tests, comprising:
- a housing area (2) configured to house a plate (P) with a plurality of wells (P') containing a sample to be analyzed;
- a processing unit (C) adapted to manage the apparatus (1); and
- at least one image detector (10) in communication with the processing unit (C) for acquiring images (Img) of the plate (P) located in the housing area (2),
wherein the processing unit (C) comprises instructions for processing the acquired images which, when executed, cause it to carry out the following steps:
- controlling the acquisition of at least one image (Img) containing at least one well (P');
- extracting a plurality of features (F) from the image (Img), said features (F) comprising at least a number of aggregates of pixels detectable in said image (Img), a number of closed contours detectable in said image (Img), and a number of elements having a determined at least partially circular and/or elliptical geometry detectable in said image (Img); and
- classifying, based on a combination of said features (F), the sample contained in the well (P'), providing information on the positivity of said sample through said classification.

2. The apparatus (1) according to claim 1, wherein the processing unit (C) is configured to assign a class to the sample contained in the well (P'), the class being selected from at least:
- a first class of negative samples, wherein a formation of a substantially uniform ring is detectable in the image (Img);
- a second class of positive samples, wherein in the image (Img) there is absence of the ring or an incomplete ring; and
- at least one third class intermediate between said first class and said second class.

3. The apparatus (1) according to claim 1 or 2, wherein the processing unit (C) is configured to identify the number of elements having a determined circular and/or elliptical geometry in the image (Img) by executing a calculation procedure based on the Hough transform for searching circles and/or on a least squares minimization for searching ellipses.

4. The apparatus (1) according to any one of the preceding claims, wherein the aggregates of pixels detectable in the image (Img) are defined as a set of contiguous dark pixels with an overall area beyond a predetermined threshold.

5. The apparatus (1) according to any one of the preceding claims, wherein the processing unit (C) is configured to calculate the number of closed contours detectable in the image (Img) having a perimeter beyond a determined threshold, and wherein said processing unit (C) is programmed to identify a closed contour, as circular, based on a pre-fixed relation between area and perimeter of said closed contour.

6. The apparatus (1) according to any one of the preceding claims, wherein the processing unit (C) is configured to identify, inside the plate (P), single wells (P') by identifying lines (Pl) corresponding to the separation lines of the strips that form said plate (P), and wherein the processing unit (C) is programmed for associating with the identified single wells (P') respective samples whose features are defined in a memory unit (MEM) thereof.

7. The apparatus (1) according to any one of the preceding claims, wherein the processing unit (C) is configured to binarize the image (Img) prior to extracting the features (F).

8. The apparatus (1) according to any one of the preceding claims, wherein the processing unit (C) is configured to obtain and process a plurality of images (Img) of a respective plurality of wells (P'), each of said wells corresponding to a specific serial dilution of the sample therein contained, thus producing as output a titration curve in relation to the sample contained in said wells (P), wherein, for each dilution, the processing unit (C) is configured to perform the classification of the sample, and wherein said processing unit (C) is programmed to automatically identify, inside the plate (P) and based on the instructions therein stored, a series of said wells (P') with serially diluted sample and analyze said wells in a consecutive way.

9. The apparatus (1) according to any one of the preceding claims, wherein the processing unit (C) is configured to perform the classification of the sample by executing a tree-decision algorithm, which is structured to define, for each of the extracted features (F), optimal threshold values in order to classify the sample, said tree-decision algorithm being trained based on example images containing formations of antigen/antibody agglomerates relating to a status of a patient.

10. The apparatus (1) according to any one of the preceding claims, wherein the processing unit (C) is configured to define, based on the combination of said features (F) extracted from the image (Img), a percentage of positivity of the sample.

11. The apparatus (1) according to any one of the preceding claims, wherein the processing unit (C) is configured to process the images (Img) acquired by means of machine-learning techniques.

12. The apparatus (1) according to any one of the preceding claims, wherein, based on an acquired image, the processing unit (C) is configured to:
- verify the actual presence of the plate (P) in the housing area (2);
- verify that said plate (P) is the plate associated with a set test; and
- verify the orientation of the plate (P).

13. The apparatus (1) according to any one of the preceding claims, comprising a door (15) which acts as a closing element of the housing area (2) and which is configured to pass from an open configuration, in which it is possible for the operator to access the housing area (2), to a closed configuration, in which it is not possible to access said housing area (2) and vice versa, said door (15) being provided with a solenoid configured to allow a magnetic closure thereof during the test.

14. The apparatus (1) according to any one of the preceding claims, wherein the processing unit (C) is programmed to perform, based on said image processing, a test of the Rapid Plasma Reagin (RPR) type.

15. The apparatus (1) according to claim 14, wherein the processing unit (C) is also programmed to perform a test of the Treponema Pallidum Haemagglutination Assay (TPHA), Widal Wright (WW) and Weil Felix (WF) type.

16. A method for performing serum agglutination tests, including the steps of:
- controlling the acquisition of at least one image (Img) containing at least one reaction well (P');
- extracting a plurality of features (F) from the image (Img), said features (F) comprising at least one number of aggregates of pixels detectable in said image (Img), a number of closed contours detectable in said image (Img), and a number of elements having a determined at least partially circular and/or elliptical geometry detectable in said image (Img); and
- classifying, based on a combination of said features (F), the sample contained in the well (P'), providing information on the positivity of said sample through said classification.
